Europäisches Patentamt

European Patent Office

Office européen des brevets

(11) Veröffentlichungsnummer: **0 024 698**

**B1**

(12) **EUROPÄISCHE PATENTSCHRIFT**

(45) Veröffentlichungstag der Patentschrift: **03.12.86**

(21) Anmeldenummer: **80104990.9**

(22) Anmeldetag: **21.08.80**

(51) Int. Cl.⁴: **A 61 B 6/03,** G 06 F 15/20

(54) **Computertomograph zur zweidimensionalen Darstellung einer Ebene eines Objektes.**

(30) Priorität: **24.08.79 US 69658**
**24.08.79 US 69943**

(43) Veröffentlichungstag der Anmeldung:
**11.03.81 Patentblatt 81/10**

(45) Bekanntmachung des Hinweises auf die
Patenterteilung:
**03.12.86 Patentblatt 86/49**

(84) Benannte Vertragsstaaten:
**DE FR GB IT**

(56) Entgegenhaltungen:
**FR-A-2 278 310**
**FR-A-2 356 160**
**FR-A-2 391 696**
**GB-A-2 002 987**
**US-A-4 126 787**

(73) Patentinhaber: **Siemens Aktiengesellschaft**
**Berlin und München**
**Wittelsbacherplatz 2**
**D-8000 München 2 (DE)**

(72) Erfinder: **Stonestrom, James Peter**
**3326 Kipling**
**Palo Alto California (US)**
· Erfinder: **Nassi, Menahem**
**2743 Waverly**
**Palo Alto California (US)**

Courier Press, Leamington Spa, England.

## Beschreibung

Die Erfindung betrifft einen Computertomographen gemäß dem Oberbegriff des Patentanspruches 1.

Bei einem bekannten Computertomographen sind eine Strahlenquelle und ein Strahlenempfänger gegenüberliegend an einem Drehrahmen befestigt, in dessen Drehachse sich der zu untersuchende Patient befindet. Die von der Strahlenquelle ausgehenden Strahlen durchdringen den zu überprüfenden Körperteil und fallen auf den Strahlendetektor, der die Absorption auf einer Vielzahl von Strahlenwegen während eines Abtastvorganges mißt. Der Drehrahmen dreht sich schrittweise um einen bestimmten Winkel um den Körper. Der Abtastvorgang wird periodisch wiederholt. Dadurch erhält man eine große Anzahl von gemessenen Detektorwerten. In einem Rechner wird die angesammelte Datenmenge verarbeitet. Die die Absorption kennzeichnenden gemessenen Detektorwerte werden miteinander korreliert und dadurch die Absorptionswerte für eine große Anzahl von Punkten innerhalb des durchstrahlten Körperteiles berechnet. Diese Absorptionswerte werden zu einer Matrix kombiniert, so daß man eine genaue Abbildung der Dichteverteilung des geprüften Körperteiles erhält.

In den US-Patentschriften 4 149 247 und 4 149 248 ist ein Computertomograph der genannten Art beschrieben, der eine fächerförmige Strahlenquelle verwendet und der mit Hilfe eines rechnerischen Faltungsverfahrens die Erzeugung des Bildes ohne Neuordnung der fächerförmigen Strahlen bewirkt, um dadurch die Fehler und die Verzögerung der Rechenzeit durch die Neuordnung in eine parallele Strahlung zu verhindern.

Weiterhin weist bei dem Computertomographen gemäß der US—PS—4 149 249, ausgehend von dem bereits beschriebenen Stand der Technik, der Rechner für die Rekonstruktion der Abbildung einen allgemeinen Arbeitscomputer, einen speziellen Arbeitscomputer und eine Steuerungslogik für das Zusammenwirken beider Arbeitscomputer auf. Diese berechnen durch Faltung und Rückprojektion der gemessenen Detektorwerte von nicht absorbierten Strahlungen die Absorptionswerte, die als ein Abbild der Dichtefunktion des Körperteils auf einer Bildwiedergabevorrichtung sichtbar gemacht werden.

Bei bekannten Computertomographen enthält der Strahlenempfänger eine Vielzahl (beispielsweise 301) von einzelnen, seitlich nebeneinander angeordneten Detektorelementen. Nach jeweils einer Drehbewegung von jeweils 1° des Drehrahmens erfolgt eine Abtastung des Strahlenempfängers, beispielsweise durch Pulsen der Strahlenquelle, so daß nach einer 360°-Drehung 360×301 gemessene Detektorwerte erhalten werden. Zur Vergrößerung der Auflösung ist es möglich, die Zahl der Detektorelemente zu erhöhen. Jedoch ist dies wegen der Abmessungen und der höheren Kosten unpraktisch.

Für eine 360°-Drehung des Drehrahmens wird eine relativ lange Zeit benötigt, während der der Patient bestrahlt wird und bewegungslos sein sollte. Um die Strahlenbelastung des Patienten und die Bewegungsunschärfe der Abbildung zu reduzieren, wurde bisher eine Zeitreduzierung der Abtastung durch Verkürzung der Drehung erreicht. Die fehlenden Detektorwerte wurden abgeschätzt oder auf Null gesetzt. Durch die fehlenden Abtastungen verschlechterten sich die Auflösung und die Genauigkeit der Rekonstruktion.

Zur Untersuchung von dynamischen Vorgängen ist es erforderlich, zur Beobachtung der gleichen Phase mehrere Ausschnitte der 360°-Drehung zur Rekonstruktion zu verwenden. Es wurden hierzu ein Verfahren und ein System vorgeschlagen, bei dem während einer oder mehrerer Drehperioden die gemessenen Detektorwerte gespeichet werden, Zur Rekonstruktion, beispielsweise eines Herzzyklus, werden mit Hilfe eines aus einem Elektrokardiogramm hergeleiteten Referenzsignales Detektorwerte von Positionswinkeln bestimmter Bewegungsphasen ausgewählt und verarbeitet. Hierbei verschlechtern sich, wie bereits erwähnt, die Bildauflösung und die Bildqualität.

Auch führen defekte Detektorelemente zur Verschlechterung der Bildqualität. Da sie entweder kein oder ein falsches Signal liefern, dürfen sie zur Rekonstruktion nicht verwendet werden, so daß sich die Anzahl der gemessenen Detektorwerte verringert. Aus der FR—A—2 356 160 ist ein Computertomograph bekannt, bei dem die Ausgangssignale eines defekten Detektors durch Korrekturwerte ersetzt werden, die durch Interpolation von Meßwerten benachbarter Detektoren erzeugt werden. Aus den Meßwerten von intakten Detektoren und den einzelnen Korrekturwerten wird dann das Bild in bekannter Weise mittels eines Faltungsprozesses berechnet. Diese umgeformten Korrekturwerte, die längs eines Strahlenpfades des defekten Detektors liegen, werden durch längs des umgekehrten Strahlenpfades liegende, umgeformte Meßwerte anderer Detektoren ersetzt.

Eine Erhöhung und Verbesserung des Auflösevermögens über das durch die Anzahl der Detektorelemente und Positionswinkel bestimmter Auflösungsvermögen hinaus wird nicht erreicht.

Der Erfindung liegt die Aufgabe zugrunde, eine Computertomographen gemäß dem Oberbegriff des Patentanspruches 1 zu schaffen, der eine Erhöhung der nutzbaren Auflösung und der Bildqualität bei gleichbleibender Anzahl der Detektorelemente und Positionswinkel ermöglicht.

Die Aufgabe wird erfindungsgemäß durch die in dem Kennzeichen des Patentanspruches 1 angegebenen Merkmale gelöst. Hierdurch erhöht sich die Anzahl der zu verarbeitenden Detektorwerte, so daß die Auflösung und die Bildqualität gesteigert werden.

Die Auflösung der Darstellung läßt sich weiter erhöhen, wenn die Schaltungsanordnung eine Interpolationsvorrichtung aufweist, die die in einem Eingangspufferspeicher enthaltenen gemessenen Detektorwerte mit in einem Speicher enthaltenen Interpolationskonstanten derart verarbeitet, daß Zwischenwerte der gemessenen Detektorwerte erhalten werden, so daß für gewünschte, weitere Positionswinkel berechnete Detektorwerte erzeugt werden.

Die Erfindung ist nachfolgend anhand eines in der Zeichnung dargestellten Ausführungsbeispiels näher erläutert. Es zeigen:

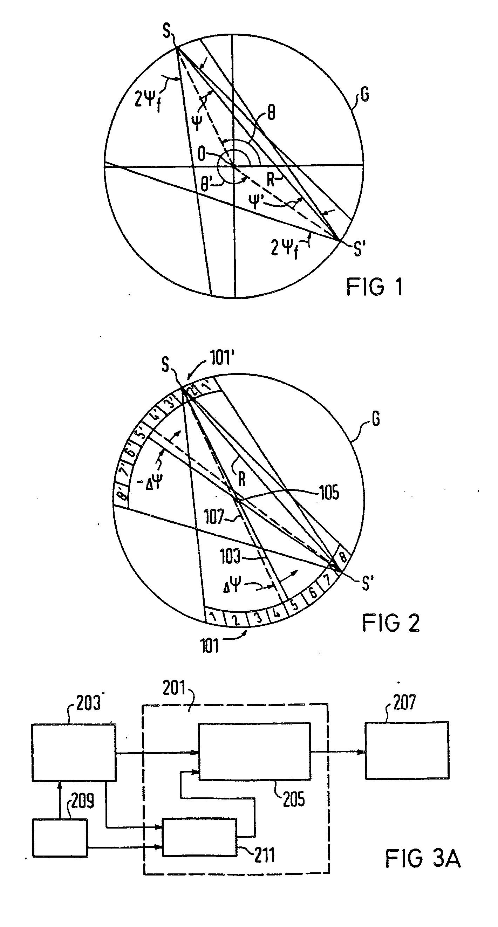

Fig. 1 eine schematische Darstellung zur Erläuterung der Reflexion,

. Fig. 2 eine schematische Darstellung der geometrischen Verhältnisse der Reflexion,

Fig. 3a und 3b Blockschaltbilder eines erfindungsgemäßen Systems,

Fig. 4 eine weitere schematische Darstellung zur Erläuterung des Verfahrens nach der Erfindung,

Fig. 5a eine schematische Darstellung der gemessenen Detektorwerte, und

Fig. 5b eine schematische Darstellung der berechneten Detektorwerte bei dem erfindungsgemäßen Verfahren und System.

In der Figur 1 ist eine schematische Darstellung der geometrischen Verhältnisse eines Computertomographiesystems mit fächerförmiger Strahlenabtastung gemäß der bereits genannten US—PS 41 49 249 abgebildet. An einem Drehrahmen G ist eine Strahlenquelle S angebracht, deren gezeichnete Stelle durch den Positionswinkel $\theta$ gekennzeichnet ist. Die Strahlenquelle S sendet ein fächerförmiges Strahlenbündel mit einem Fächerwinkel von $2\psi_f$ aus. Auf der der Strahlenquelle S gegenüberliegenden Seite des Drehrahmens G ist ein nicht dargestellter Strahlenempfänger angebracht, der die gemessenen Detektorwerte erzeugt. Ein von der Strahlenquelle S ausgehender Strahl R erscheint gegenüber der Symmetrieachse des Fächers unter einem Strahlenwinkel $\psi$. Der Strahl R trifft auf dem Drehrahmen G an einen Punkt S' auf. Die gemessenen Detektorwerte dieses Strahles R können durch $L(\theta,\psi)$ beschrieben werden. Den gleichen Meßwert würde eine im Punkt S' befindliche Strahlenquelle erzeugen, die die Koordinaten $(\theta',\psi')$ aufweist. Hierdurch ergibt sich eine Redundanz, die bei dem vorliegenden Anmeldungsgegenstand erfindungsgemäß ausgenutzt werden soll. Die Zusammenhänge der Winkel lassen sich durch folgende Gleichungen ausdrücken:

(1a) $$\theta'=\theta+\pi+2\psi$$

(1b) $$\psi'=-\psi$$

Aus diesen Gleichungen lassen sich die Beziehungen für die beiden äquivalenten Messungen des betrachteten Strahles R ableiten:

(2) $$L(\theta',\psi')=L(\theta+\pi+2\psi,-\psi)$$

Aus den Gleichungen (1) und (2) lassen sich durch die gemessenen Detektorwerte $L_M(\theta,\psi)$ die reflektierten Detektorwerte $L_R(\theta',\psi')$ berechnen:

(3) $$L_R(\theta',\psi')=L_M(\theta,\psi)$$

und

$$L_R(\theta',\psi')=L_M(\theta'-\pi+2\psi',-\psi')$$

Aus der Gleichung (3) folgt, daß durch die Reflexion die berechneten Detektorwerte $(L_R(\theta',\psi')$ bei einem Positionswinkel $\theta'$ aus den gemessenen Detektorwerten

$$L_M(\theta'-\pi+2\psi',-\psi')$$

für alle Strahlenwinkel

$$\psi'(-\psi_f\leq\psi'\leq\psi_f)$$

bestimmt werden können. Hieraus ist auch ersichtlich, daß zur Berechnung eines ganzen reflektierten Strahlenflächers gemessene Detektorwerte erforderlich sind, die den zweifachen Fächerwinkel umfassen.

Die durch die Abstastung erzeugten Detektorwerte werden bei Positionswinkeln im Abstand von beispielsweise $\Delta\theta=1°$ erzeugt. Die Auflösung bei jeder Abtastung beträgt beispielsweise $\Delta\psi=0,11°$. Das bedeutet, daß der Strahlenwinkel ungefähr 10 mal feiner als der Positionswinkel ist. Für die Berechnung des reflektierten Detektor-wertes $L_R(\theta',\psi')$ muß man nach Gleichung (3) einen Detektorwert

$$L_M(\theta'-\pi+2\psi',-\psi')$$

bei einem Strahlenwinkel von $\psi'$ gemessen haben. Jedoch existiert für $\psi'\neq0$ nicht unbedingt ein gemessener Detektorwert. Also muß zur Berechnung von $L_R$ der zugehörige, nicht existierende Detektorwert $L_M(\theta,\psi)$ gebildet werden. Dies kann durch Interpolation zwischen zwei aufeinanderfolgenden Positionen der Strahlenquelle erfolgen.

In der Figur 2 ist wieder der Drehrahmen G schematisch dargestellt. Gegenüber der Strahlenquelle S ist ein Strahlenempfänger 101 angeordnet, der z.B. 301 einzelne Detektorelemente enthält. Zum besseren Verständnis sind hier jedoch nur acht Detektorelemente gezeigt. Von einem Punkt S' aus ist ein fächerförmiges reflektiertes Strahlenbündel dargestellt, das auf einen nicht vorhandenen, reflektierten Strahlenempfänger 101' fällt, der ebenfalls acht Detektorelemente enthält.

Eine Mittellinie 103 geht von der Strahlenquelle S durch die den Mittelpunkt der Anordnung bildenden Drehachse 105. Das geometrische Zentrum des Strahlenempfängers ist hierzu seitlich versetzt. Die Symmetrieachse 107 vom Strahlenfächer reicht von der Strahlenquelle S bis zur Mitte des Strahlenempfängers 101, liegt also in der Figur 2 zwischen den Detektorelementen 4 und 5. Dieser Versatz um einen Winkel Δψ verbessert die räumliche Auflösung des Systems. Also Folge einer solchen Anordnung ist der Weg Strahlenquelle - Detektorelement bei jedem Positionswinkel verschieden. Durch die Berechnung von Detektorwerten erhöht sich somit die Bildauflösung. Durch die Reflexion erhält man ebenfalls einen gleich großen Versatz der reflektierten Detektorelemente 101' um den Versatzwinkel −Δψ.

In der Figur 3A ist ein Blockschaltbild eines erfindungsgemäßen computertomographischen Systems dargestellt. Diese System weist eine Meßvorrichtung 203 auf, die beispielsweise aus der Strahlenquelle, dem Strahlenempfänger und Meßverstärkern besteht. Die gemessenen Detektorwerte werden einem Rechner 201 zugeführt, der die Berechnung der Darstellung durchführt, die auf einer nachgeschalteten Bildwiedergabevorrichtung 207 angezeigt wird. Der Rechner 201 enthält einen Rekonstruktor 204 und eine Reflexionslogik 211. Eine Steuervorrichtung 209 koordiniert die Meßvorrichtung 203 und die Reflexionslogik 211, die ebenfalls mit dem gemessenen Detektorwerten beaufschlagt wird. Der Ausgang der Reflexionslogik 211 ist mit dem Rekonstruktor 205 verbunden.

Die Meßvorrichtung 203 erzeugt bei jeder Abtastung 301 Detektorwerte. Bei einer gesamten Drehung erhält man also 360 solcher Datensätze. Durch die Reflexion ist es aber nicht nötig, eine gesamte Drehung zur Abtastung vorzusehen. Beispielsweise können auch Detektorwerte nur von einem 215°-Meßbereich (180°- und 35°-Fächerwinkel) erzeugt werden, so daß dem Rechner 201 in diesem Falle nur 215×301 gemessene Detektorwerte zugeführt werden.

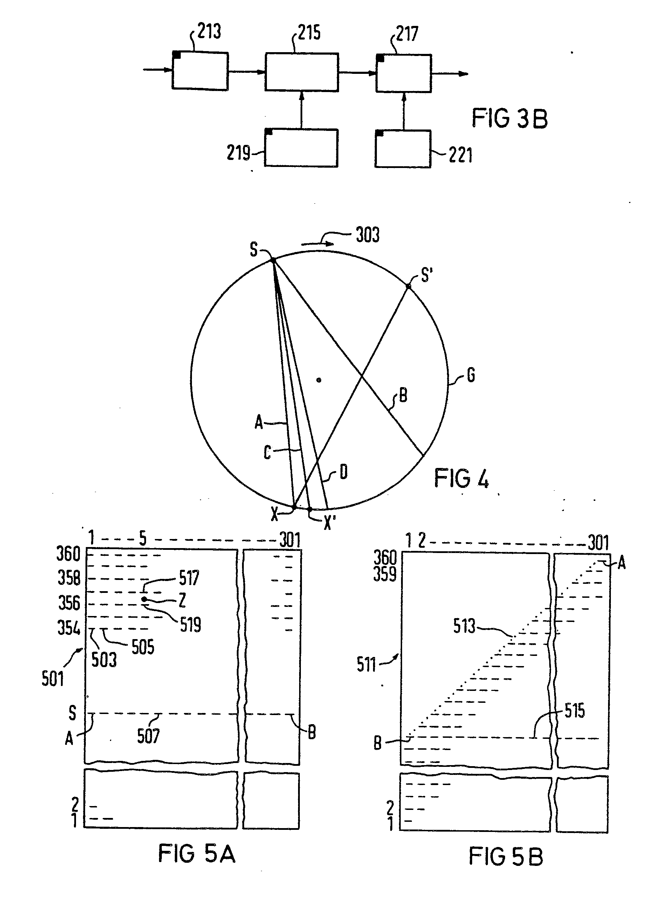

In der Figur 3B ist die Reflexionslogik 211 dargestellt. Sie weist einen Eingangspufferspeicher 213 auf, der mit der Meßvorrichtung 203 verbunden ist und dem die gemessenen Detektorwerte zugeführt werden. Dieser Speicher 213 kann beispielsweise aus einem RAM bestehen. In einer Interpolationsvorrichtung 215 werden die Detektorwerte aufgrund der in einem Speicher 219 (RAM oder ROM) enthaltenen Interpolationskonstanten verarbeitet. Die interpolierten Werte werden in Speicherplätze eines Ausgangspufferspeichers 217 eingelesen, die durch Verschiebungskonstanten, die in einem Speicher 221 gespeichert sind, ausgewählt werden. Diese Verschiebungskonstanten sortieren die reflektierten Werte nach den Gleichungen (1a) und (1b). Der Ablauf des Datenflusses innerhalb der Reflexionlogik 211 wird durch die Steuervorrichtung 209 bewirkt. Die hierzu benötigten Steuerleitungen, die die Steuervorrichtung 209 mit den einzelnen Blöcken 213 bis 221 verbinden, sind zur besseren Übersicht nicht dargestellt.

Die Speichermittel der Reflexionslogik 211 dienen dazu, die anliegenden gemessenen Detektorwerte zu erfassen, damit sie verarbeitet werden können. Die berechneten Detektorwerte werden ebenfalls gespeichert, bis sie von dem Rekonstruktor 205 zur Bilderzeugung benötigt werden.

So können beispielsweise zur Darstellung einer bestimmten Phase eines Herzzyklus die gemessenen Detektorwerte einer 360°-Drehung in dem Eingangspufferspeicher 213 gespeichert sein. Nach einem eingangs bereits erwähnten Verfahren können nach erfolgter Abtastung die Detektorwerte ausgewählt werden, die der gewünschten Phase des Herzzyklus entsprechen. Diese Auswahl kann durch ein Referenzsignal getroffen werden, das von Impulsen aus einem Elektrokardiogramm abgeleitet wird. Bei dieser Auswahl fehlen Abtastungen bei vielen Positionswinkeln. Durch die vorliegende Erfindung werden jedoch in der Reflexionslogik einige der verlorengegangenen Detektorwerte ersetzt und dem Rekonstruktor 205 zugeführt, so daß eine Bildwiedergabe mit hoher Auflösung ermöglicht wird.

Die gemessenen Detektorwerte von der Meßvorrichtung 203 brauchen nicht in einem Speicher zwischengespeichert zu werden. Sie können auch durch die Reflexionslogik 211 im On-line-Betrieb verarbeitet werden, d.h., daß beispielsweise bei der soeben beschriebenen Herzuntersuchung die gewünschten Detektorwerte sofort ausgewählt und mit anderen Werten zusammengefaßt werden.

Anstelle der bereits beschriebenen Reduktion der Detektorwerte kann die Erfindung auch auf eine oder mehrere 360°-Drehung(en) angewendet werden. Durch die Reflexion erhöht sich die in dem Rechner 201 verarbeitete Datenmenge, wodurch die Bildauflösung und Bildqualität derart ansteigen, als ob man eine höhere Anzahl von Detektorelementen verwendet hätte.

Gemäß einer Weiter bildung der vorliegenden Erfindung ist es zweckmäßig, zur Reflexion Punkte zu verwerten, die nicht den Positionen der gemessenen Detektorwerte entsprechen, sondern seitlich von ihnen versetzt sind. Dies wird durch die Interpolation zwischen aufeinanderfolgenden gemessenen Detektorwerten eines Detektors erreicht. Zur Erhöhung der Bildauflösung werden diese Werte nach Gleichung (3) reflektiert. Hierzu werden die gemessenen Detektorwerte von jedem Positionswinkel in einem Speicher, der in der Meßvorrichtung 203 enthalten sein kann, eingelesen und mit den durch die Reflexion berechneten Detektorwerten eines korrespondierenden Positionswinkels verknüpft. Diese verknüpften Daten werden dem Rekonstruktor 205 zugeführt, so daß ihm Werte entsprechend einer höheren Elementendichte zur Verarbeitung zur Verfügung stehen.

In einer anderen Arbeitsweise werden die gemessenen Detektorwerte von allen Positionswinkeln mit Nullen verknüpft und dem Rekonstruktor 205 zugeführt, als ob sie von einem Strahlenempfänger mit einer höheren Elementendichte kommen würden. Ähnlich werden die reflektierten berechneten Detektorwerte eines jeden gemessenen Positionswinkels mit Nullen verknüpft und dem Rekonstruktor 205 zugeführt.

Da der Rekonstruktor 205 die ihm zugeführten Daten linear verarbeitet, ist diese Verfahren voll äquivalent zu den bisher beschriebenen. In einigen Fällen kann die Verknüpfung der Nullen durch eine einfache Änderung des Rekonstruktors 205 erreicht werden.

In der Figur 4 ist ähnlich wie in der Figur 1 eine schematische Darstellung der geometrischen Verhältnisse abgebildet. Auf dem Drehrahmen G ist die Strahlenquelle S angeordnet, die ein fächerförmiges Strahlenbündel aussendet, das durch die Randstrahlen A, B begrenzt wird. Die Strahlenquelle S wird bei der Abtastung in Richtung des Pfeiles 303 jeweils um 1° weitergedreht. Weist nun der Strahlenempfänger 301 Detektorelemente auf und wird bei der Abtastung der Drehrahmen G mit der Strahlenquelle S und dem Strahlenempfänger 101 um 360° gedreht, so erhält main einen Datensatz, bestehend aus 360×301 gemessenen Detektorwerten.

Diese Werte sind schematisch in der Figur 5A dargestellt. Sie bilden zusammen einen Datensatz 501. Die Zahlen der Detektorelemente 1 bis 301 erscheinen am oberen Rand des Datensatzes 501; die Zahlen der Positionswinkel 1 bis 360 sind am linken Rand des Datensatzes angegeben. Die Detektorwerte von aufeinanderfolgenden Detektorelementen erzeugen bei jedem Winkel eine Zeilen von gemessenen Detektorwerten 503, 505 usw. in dem Datensatz 501. Die zeitlich nacheinander erfolgten Messungen eines Detektorelementes, beispielsweise des Elementes 5, sind in einer Spalte untereinander angeordnet.

Während einer Abtastung bei einem Positionswinkel erzeugt die Strahlenquelle ein fächerförmiges Strahlenbündel, das durch die Randstrahlen A und B begrenzt wird. Dies entspricht in der Figur 5A einer Zeile bei einer Position der Strahlenquelle S. Die gemessenen Detektorwerte der Randstrahlen A und B erscheinen an den Endpunkten der Zeile 507.

Werden nun die gesamten gemessenen Detektorwerte des Datensatzes 501 erfundungsgemäß reflektiert, so erhält man einen reflektierten Datensatz 511, der in der Figur 5B dargestellt ist. In diesem reflektierten Datensatz 511 erscheint die Abtastung bei einer Position der Strahlenquelle S, die in dem Datensatz 501 die Zeile 507 bildet, in einer Diagonalen 513, deren Endpunkte wiederum die Detektorwerte A und B der Randstrahlen sind. Aus den Figuren 4 und 5 ist ersichtlich, daß zur Berechnung auf der Basis einer reflektierten Strahlenquelle mit fächerförmigen Strahlenbündel bei einem Punkt X es notwendig ist, die Strahlenquelle S von ihrer Position in Richtung des Pfeiles 303 zur Position der Strahlenquelle S' zu drehen. Das bedeutet aber, daß zur Erzeugung eines reflektierten Fächers bei einem Punkt X Messungen durchgeführt werden müssen, die, wie bereits erwähnt, den doppelten Fächerwinkel $2 . (2\psi_f)$ überstreichen müssen.

In dem reflektierten Datensatz 511 wird dies durch die Zeile 515 gekennzeichnet. Damit man eine vollständige Zeile 515 erhält, müssen also genügend Diagonalen 513 erzeugt werden. In diesem Datensatz 511 kennzeichnet der vertikale Abstand zwischen B und A den doppelten Fächerwinkel der Quelle S.

In der Figur 4 sind noch zwei weitere Strahlen C und D innerhalb des fächerförmigen Strahlenbündels dargestellt. Der Strahl C trifft auf den Strahlenempfänger 101 bei einem Punkt X' auf. Dieser Punkt X' kann erfindungsgemäß als reflektierte Strahlenquelle betrachtet werden. Jedoch kann bedingt durch die geometrische Aufteilung der Abtastung eine Reflexion an einem Punkt X' gewünscht werden, für den keine für die Berechnung benötigten gemessenen Detektorwerte vorliegen. Um dennoch bei diesem Punkt X' eine Reflexion durchführen zu können, werden die gemessenen Daten eines Detektors von aufeinanderfolgenden Positionswinkeln $\theta$ interpoliert. Dies ist anhand des Detektorelementes 5 in Figur 5A erläutert. Bei einem Positionswinkel $\theta$ von 357° wird ein Detektorwert 517 erhalten und bei einem Positionswinkel von 356° ein Detektorwert 519. Um die Reflexion auf einen Positionswinkel zwischen beiden Winkeln durchführen zu können, werden die gemessenen Detektorwerte 517 und 519 interpoliert und man erhält den Zwischenwert Z.

Zur Erhöhung der Bildauflösung kann die Interpolation auch zwischen benachbarten Detektorelementen eines Positionswinkels erfolgen. Dadurch erhält man zur Berechnung der reflektierten Detektorwerte interpolierte Werte, die zu Positionen zugeordnet sind, die im Hinblick auf die den gemessenen Detektorwerten zugeordneten Positionen seitlich versetzt erschienen. Sind die Detektorelemente, wie bereits beschrieben, seitlich um ein Viertel einer Elementenbreite gegenüber der Mittellinie 103 versetzt, sollte die Versetzung der Hälfte der Breite der Detektorelemente entsprechen. Dadurch erhält man eine Reflexion, die ebenfalls gegenüber der Mittellinie um ein Viertel der Elementenbreite versetzt ist.

Die Erfindung ist anhand eines drehenden CT-Systems mit einer Strahlenquelle zur Erzeugung eines fächerförmigen Strahlenbündels beschrieben. Die Erfindung ist aber auf jedes andere CT-System ebenfalls anwendbar, das eine Strahlenquelle zur Erzeugung durchdringender Strahlen und einen Strahlenempfänger zur Messung der Transmissionswerte divergierender Transmissionswege in einer Ebene enthält.

**Patentansprüche**

1. Computertomograph zur zweidimensionalen Darstellung einer Ebene eines Objektes mit einer Vorrichtung mit einer Strahlenquelle (S) zur Erzeugung eines fächerförmigen Strahlenbündels und mit einem Strahlenempfänger (101), der in einer Reihe angeordnete Detektorelemente (1 bis 8) aufweist, die in einer Ebene mit der Strahlenquelle (S) dieser gegenüberliegen und zum Erfassen der nicht von dem Objekt absorbierten Strahlung dienen, wobei die Vorrichtung relativ zu dem Objekt durch eine schrittweise Drehung um eine Drehachse (105) verlagerbar ist, so daß zwischen den Drehschritten vom Strahlenempfänger (101) bei einer Vielzahl von Positionswinkeln (θ) Sätze von gemessenen Detektorwerten erhalten werden, die die zweidimensionale Abbildung des Objektes durch Verarbeitung der Detektorwerte in einem Rechner ermöglichen, der mit einer Bildwiedergabevorrichtung (207) zur Darstellung der verarbeiteten Detektorwerte verbunden ist, dadurch gekennzeichnet, daß eine Schaltungsanordnung (211) (Reflexionslogik) vorgesehen ist, deren Eingang mit den Sätzen von gemessenen Detektorwerten beaufschlagt ist und die hieraus Sätze von berechneten Detektorwerten für gewünschte, weitere Positionswinkel (θ') erzeugt, indem der gemessene Detektorwert, der durch einen von der Strahlenquelle (S) ausgehenden Strahl (R) in einem Detektorelement (1 bis 8) hervorgerufen ist, demjenigen gedachten Detektorelement (1' bis 8') zugeordnet wird, auf das bei einem gedachten umgekehrten (reflektierten) Verlauf des Strahles (R) und somit inversen Anordnung von Strahlenquelle (S') und Strahlenempfänger (101') der Strahl (R) treffen würde, und daß die das Ausgangssignal der Reflexionslogik (211) bildenden berechneten Detektorwerte und die gemessenen Detektorwerte dem Rechner zugeführt sind, der die gemessenenen und die berechneten Detektorwerte verarbeitet.

2. Computertomograph nach Anspruch 1, dadurch gekennzeichnet, daß die Reflexionslogik (211) eine Interpolationsvorrichtung (215) aufweist, die die in einem Eingangspufferspeicher (213) enthaltenen gemessenen Detektorwerte mit in einem Speicher (219) enthaltenen Interpolationskonstanten derart verarbeitet, daß Zwischenwerte der gemessenen Detektorwerte erhalten werden, so daß für gewünschte, weitere Positionswinkel (θ') berechnete Detektorwerte erzeugt werden.

**Revendications**

1. Tomodensitomètre servant à réaliser la représentation bidimensionelle d'un plan d'un objet, comportant un dispositif muni d'une source de rayonnement (S) servant à produire un faisceau de rayonnement en forme d'évantail et un récepteur de rayonnement (101), qui comporte des éléments détecteurs (1 à 8) disposés selon une rangée et qui sont situés en vis-à-vis d'une source de rayonnement (S), dans un plan contenant cette dernière, et servent à détecter le rayonnement qui n'est par absorbé par l'objet, le dispositif pouvant être déplacé par rapport à l'objet grâce à une rotation pas-à-pas autour d'un axe de rotation (150), de telle sorte qu'entre les pas de rotation le récepteur de rayonnement (101) délivre pour une multiplicité d'angles de positions (θ), des ensembles de valeurs mesurées par les détecteurs et qui permettent la représentation imagée bidimensionnelle de l'objet par traitement des valeurs des détecteurs dans un calculateur qui est relié à un dispositif (207) de reproduction d'images, servant à représenter les valeurs traitées des détecteurs, caractérisé par le fait qu'il est prévu un montage (211) (logique à réflexion), dont l'entrée est chargée par les ensembles de valeurs mesurées des détecteurs et qui produit, à partir de là, des ensembles de valeurs calculées des détecteurs pour d'autres angles de position désirés (θ'), par le fait que la valeur mesurée du détecteur, que est provoquée par un faisceau (R) partant de la source de rayonnement (S), est associée à l'élément détecteur imaginaire (1' à 8') sur lequel le faisceau (R) tomberait dans le cas d'un parcours imaginaire inverse (réfléchi) du rayon (R) et par conséquent dans le cas d'un montage inverse de la source de rayonnement (S') et du récepteur de rayonnement (101'), et que les valeurs calculées des détecteurs, constituant le signal de sortie de la logique à réflexion (211), et les valeurs mesurées des détecteurs sont envoyés au calculateur qui traite les valeurs mesurées des détecteurs et des valeurs calculées des détecteurs.

2. Tomodensitomètre suivant la revendication 1, caractérisé par le fait que la logique à réflexion (211) comporte un dispositif d'interpolation (215) qui traite les valeurs mesurées du détecteur, contenues dans une mémoire tampon (213), avec des constantes d'interpolation contenues dans une mémoire (219) de manière que l'on obtient des valeurs intermédiaires des valeurs mesurées des détecteurs, de sorte que l'on produit des valeurs calculées des détecteurs pour d'autres angles désirés de positions (θ').

**Claims**

1. A computer tomograph for the two-dimensional representation of a plane of an object with a device having a radiation source (S) for producing a fan-shaped beam and a radiation receiver (101) which has detector elements (1 to 8) arranged in a row flush with the radiation source (S) opposite the latter to detect radiation not absorbed by the object, where the device can be displaced relative to the object by a gradual rotation about an axis of rotation (105) such that, between the rotary steps, sets of measured detector values are received from the radiation receiver (101) for a plurality of position angles (θ) so as to allow two-dimensional representation of the object by processing the detector values in a computer connected to an image reproduction device (207) for representing the processed detector values, characterised in that a circuit arrangement (211) (reflection logic) is provided, whose input is acted upon by the sets of measured detector values and which produces sets of computed detector values for required, further position angles (θ') therefrom, in that the measured detector value which is caused in a detector element (1 to 8) by a beam-component (R) which emanates from the radiation source (S) is assigned to that imaginary detector element (1' to 8') which would be hit by the beam-component (R) in the event of an imaginary reversed (reflected) course of the beam-component (R), and thus inverse arrangement of the radiation source (S′) and radiation receiver (101′), and that the computed detector values which form the output signal of the reflection logic (211) and the measured detector values are supplied to the computer which processes the measured and the computed detector values.

2. A computer tomograph as claimed in Claim 1, characterised in that the reflection logic (211) has an interpolation device (215) which processes the measured detectors contained in an input buffer store (213) by means of interpolation constants contained in a store (219), in such a manner that intermediate measured detector values are obtained, so that detector values which are computed for required, further position angles (θ′), are produced.

FIG 1

FIG 2

FIG 3A

1

FIG 3B

FIG 4

FIG 5A

FIG 5B